# FASCICULE DE BREVET EUROPEEN

(11) **EP 3 054 859 B1**
(45) Date de publication et mention de la délivrance du brevet: **15.01.2020**
(21) Numéro de dépôt: 14796185.8
(22) Date de dépôt: 24.09.2014
(51) Int. Cl.: A61B 17/04, A61B 17/34, A61B 17/00

(54) **DISPOSITIF POUR REFERMER UN ORIFICE D'ACCÈS D'UN INSTRUMENT CHIRURGICAL FORMÉ D'UNE PAROI**
VORRICHTUNG ZUM VERSCHLIESSEN EINER ZUGANGSÖFFNUNG EINES CHIRURGISCHEN INSTRUMENTS, GEBILDET DURCH EINE WAND
DEVICE FOR CLOSING AN ACCESS ORIFICE OF A SURGICAL INSTRUMENT FORMED BY A WALL

(30) Priorité: 09.10.2013 FR 1359782
(43) Date de publication de la demande: 17.08.2016
(73) Titulaire: Wames, 42160 Saint Cyprien (FR); CHU de Saint-Etienne, 42055 Saint-Etienne Cedex 2 (FR); CMI'NOV, 43120 Monistrol sur Loire (FR)
(72) Inventeur: DUPREY, Ambroise, F-42270 Saint Priest en Jarez (FR); PAIN, Bernard, F-43120 Monistrol Sur Loire (FR); WIECEK, William, F-42160 Saint Cyprien (FR)
(74) Mandataire: Cabinet Laurent & Charras
(86) Numéro de dépôt international: PCT/FR2014/052389
(87) Numéro de publication internationale: WO 2015/052400

(56) Documents cités:
- US-A1- 2006 069 397
- US-A1- 2008 033 459
- US-A1- 2010 114 110

## Description

L'invention concerne un dispositif pour refermer un orifice d'accès d'un instrument chirurgical.

L'invention trouve une application particulièrement avantageuse dans le cas d'une cœlioscopie qui, de manière connue, est une technique chirurgicale mini invasive de diagnostic et/ou d'intervention qui peut être utilisée sur la cavité abdominale, l'appareil digestif ou autre type d'intervention notamment cardiaque.

De manière parfaitement connue pour un homme du métier, une cœlioscopie consiste donc à accéder à une cavité abdominale par exemple, sans ouvrir la paroi abdominale.

Pour l'essentiel, pour réaliser une cœlioscopie, on utilise un système optique introduit dans la cavité abdominale au travers d'une cicatrice pratiquée par le chirurgien. Un système optique est relié à un système informatique pour être positionné par le chirurgien pendant l'opération. Du gaz carbonique est introduit dans la cavité abdominale de sorte que la pression exercée par le gaz soulève la paroi abdominale créant ainsi un espace où le chirurgien peu regarder et où il peut introduire les instruments chirurgicaux nécessaires en fonction du type d'opération à effectuer. Des trocarts sont introduits à travers la paroi ainsi soulevée pour permettre au chirurgien de passer au travers desdits trocarts, des instruments de différents types, de différents diamètres en fonction du geste opératoire à effectuer A titre indicatif, les trocarts peuvent présenter un diamètre variant de 5 à 12 mm environ.

Cette technique est notamment illustrée dans la demande US 2008/033459.

Après avoir réalisé cette opération sous cœlioscopie, les différents instruments, y compris le trocart, sont retirés de sorte qu'il est ensuite nécessaire au chirurgien de refermer l'orifice au travers duquel a été introduit le trocart notamment. Une telle fermeture est obligatoire notamment lorsque le trocart présente un diamètre supérieur à 8 mm et doit être réalisée avec un grand soin afin d'éviter tout problème post-opératoire. Généralement, la fermeture de l'orifice est effectuée au moyen d'aiguilles courbes équipées d'un fil de suture. L'aiguille est engagée au travers de la paroi à partir de l'un des côtés de l'orifice puis retirée de l'autre côté de manière à réaliser une boucle et former un nœud.

Cette opération de nouage est particulière délicate à réaliser étant donné qu'elle est effectuée en aveugle, le système de vision ayant déjà été retiré.

L'invention s'est fixée pour but de remédier à ces inconvénients de manière simple, sûre, efficace et rationnelle.

Le problème que se propose de résoudre l'invention est de parfaitement sécuriser cette opération de fermeture en utilisant le trocart et le système de vision afin que le chirurgien puisse parfaitement visualiser la suture en vue de réaliser la fermeture de l'orifice.

Pour résoudre un tel problème, il a été conçu et mis au point un dispositif selon la revendication 1.

Il résulte de ces caractéristiques, qu'une fois l'opération terminée, l'introduction du corps tubulaire dans le trocart, permet de placer un point de suture suffisant pour fermer le trou initialement créé pour l'introduction du trocart. La suture est positionnée de manière symétrique de part et d'autre du trou de l'incision, à une distance suffisante afin de sécuriser la fermeture.

De manière avantageuse dans une forme de réalisation, les bras sont articulés à l'embout pour occuper une position et rabattus dans des logements formés d'une manière diamétralement opposée selon les génératrices dudit embout et occuper une position d'alignement linéaire de part et d'autre de l'embout correspondant à la saisie du fil..

Pour résoudre le problème posé de permettre aux aiguilles d'occuper une position d'intégration à l'intérieur du corps et une position repliée angulairement à l'intérieur du corps, les aiguilles sont réalisées dans un alliage super élastique.

Selon d'autres caractéristiques, les bras sont assujettis à un organe de commande actionnable à partir de l'extérieur du corps, pour occuper à volonté la position rabattue ou la position écartée en alignement linéaire. Les extrémités des bras présentent des fentes aptes à retenir les brins du fil en position écartée et à coopérer avec les aiguilles pour saisir lesdits brins et à libérer ledit fil en position rabattue. Les aiguilles sont assujetties à un organe de commande actionnable à partir de l'extrémité du corps pour être déplacées linéairement selon deux sens opposés correspondant soit au déplacement angulaire desdites aiguilles soit, à leur rétractation à l'intérieur dudit corps.

Selon une forme de réalisation, l'embout cylindrique recevant le fil de suture est démontable.

Pour résoudre le problème posé d'assurer la commande des bras et des aiguilles et de permettre le positionnement du corps tubulaire par rapport au trocart, les organes de commande des bras et des aiguilles sont montés dans une tête de manœuvre formée coaxialement au corps et présentant une portée axiale de centrage et de butée coopérant avec une portée femelle complémentaire que présente une extrémité du trocart considérée à l'opposé de son extrémité d'introduction.

Compte tenu de ces caractéristiques, la fermeture du fil d'accès résultant de la cœlioscopie est particulièrement sûre et efficace s'effectue de la manière suivante:
- on introduit le corps du trocart et on enfonce le corps pour que son extrémité déborde du trocart, les bras étant rabattus,
- on écarte les bras,
- on déplace le corps pour faire plaquer les bras écartés contre la paroi,
- on déplace les aiguilles qui perforent la paroi de part et d'autre de l'orifice et, d'une manière angulaire et symétrique, les aiguilles accrochant le fil porté par les bras,
- on retire les aiguilles qui déroulent le fil, lesdites aiguilles se repositionnant à l'intérieur du corps en maintenant le fil dans le trocart,
- on repousse le corps et on escamote les bras pour former une boucle,
- on retire l'ensemble trocart-corps pour pouvoir saisir les brins ancrés dans la paroi abdominale et faire un nœud.

L'invention est exposée ci-après plus en détail à l'aide des figures des dessins annexés dans lesquels:
- La figure 1 est une vue en perspective du corps tubulaire pour la mise en place dans le trocart.

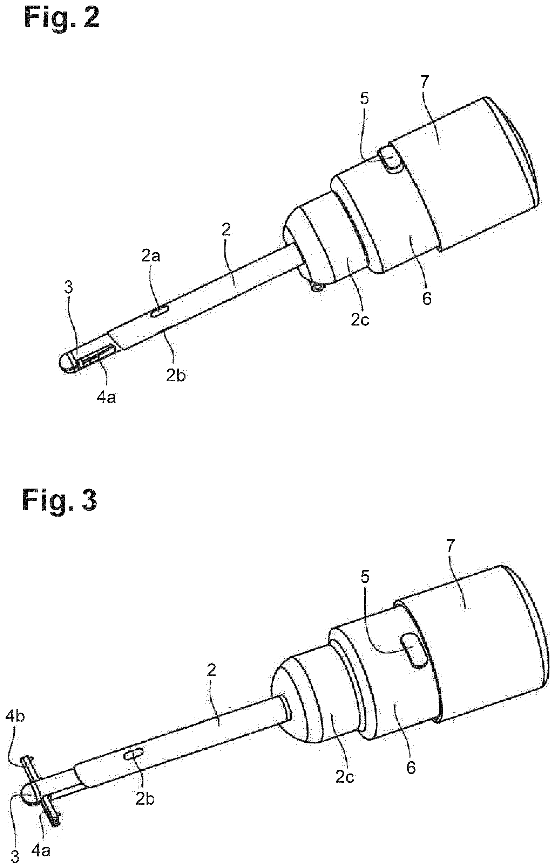

- La figure 2 est une vue en perspective montrant la mise en place du corps tubulaire dans le trocart en position de butée.
- La figure 3 est une vue en perspective du trocart en position de butée et après écartement des bras.

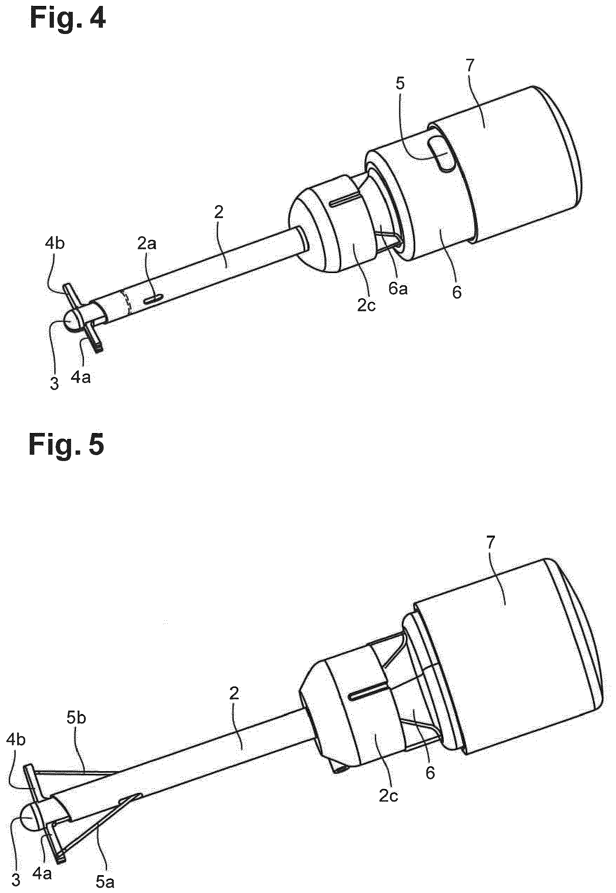

- La figure 4 est une vue en perspective correspondant à la figure 3 après dégagement partiel du corps tubulaire par rapport au trocart.
- La figure 5 est une vue en perspective de l'appareil introduit dans le trocart selon la position illustrée figure 4 mais après déploiement des aiguilles en position de coopération avec les extrémités des bras pour saisir le fil.
- La figure 6 est une vue en perspective correspondant à la figure 5 montrant la rétraction des aiguilles à l'intérieur du corps tubulaire et au travers du trocart pour la traction du fil porté par les bras.
- La figure 7 est une vue correspondant à la figure 6 en position rentrée des aiguilles.
- La figure 8 est une vue partielle en perspective à plus grande échelle montrant la formation de la boucle.
- La figure 9 est une vue en coupe du corps tubulaire.
- Les figures 10 à 17 montrent les différentes séquences du mode opératoire pour la réalisation du nœud en vue de la fermeture de l'orifice d'introduction.

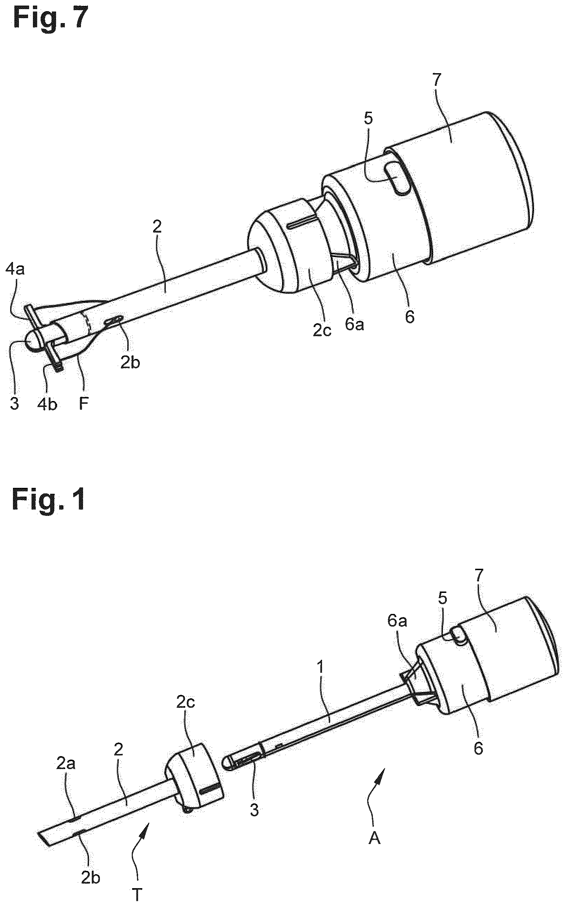

Comme le montre notamment la figure 1, le dispositif selon l'invention comprend un trocart (T) et un appareil (A) apte à coopérer avec ledit trocart et à permettre la mise en place d'un fil de suture (F) par rapport à un orifice (O) qui doit être refermé.

L'appareil (A) présente un corps tubulaire allongé (1) apte à être engagé, avec capacité de coulissement, à l'intérieur d'une canule (2) du trocart (T). La longueur du corps tubulaire (1) est déterminée pour pouvoir déborder de la canule (2) du trocart engagé dans l'orifice à refermer.

L'extrémité du corps tubulaire (1) destinée à être engagée dans le trocart (T) présente des moyens aptes à former, de part et d'autre de l'orifice (O) d'introduction et dans l'épaisseur de la paroi abdominale (P), des canaux (c) orientés angulairement et symétriquement par rapport audit orifice, d'une part, et à saisir au moins un fil de suture (F) conditionné dans un embout (3) que présente ladite extrémité du corps tubulaire (1), d'autre part.

Ces moyens sont constitués par des bras escamotables (4a) et (4b) coopérant avec le fil de suture (F) et par des aiguilles escamotables (5a) et (5b) aptes à saisir ledit fil. Le fil (F) est enroulé dans l'embout (3) afin de sortir dudit embout par un orifice et coopérer avec les bras (4a) et (4b) afin d'être saisi par les aiguilles (5a) (5b) comme il sera indiqué dans la suite de la description.

Les bras (4a) et (4b) sont articulés dans l'embout (3) afin d'occuper soit, une position rabattue soit, une position écartée. En position rabattue, les bras (4a) et (4b) coopèrent avec des logements formés de manière diamétralement opposé selon les génératrices de l'embout (3). En position écartée, les bras sont disposés en alignement linéaire de part et d'autre de l'embout (3), cette position correspondant à la saisie dudit fil comme il sera indiqué ci-après.

Les bras (4a) et (4b) sont assujettis à un organe de commande (5) actionnable à partir de l'extérieur du corps (1). Plus particulièrement, l'organe de commande (5) est monté dans une tête de manœuvre (6) formée coaxialement au corps (1) à l'opposé de l'embout (3). Par exemple l'organe de commande (5) agit sur un système du type crémaillère afin de permettre au bras (4a) et (4b) d'occuper à volonté la position rabattue ou la position écartée en alignement linéaire comme indiqué.

Les extrémités des bras (4a) et (4b) présentent des fentes (4a1) et (4b1) aptes à retenir les brins du fil (F) en position écartée desdits bras et à libérer le fil en position rabattue.

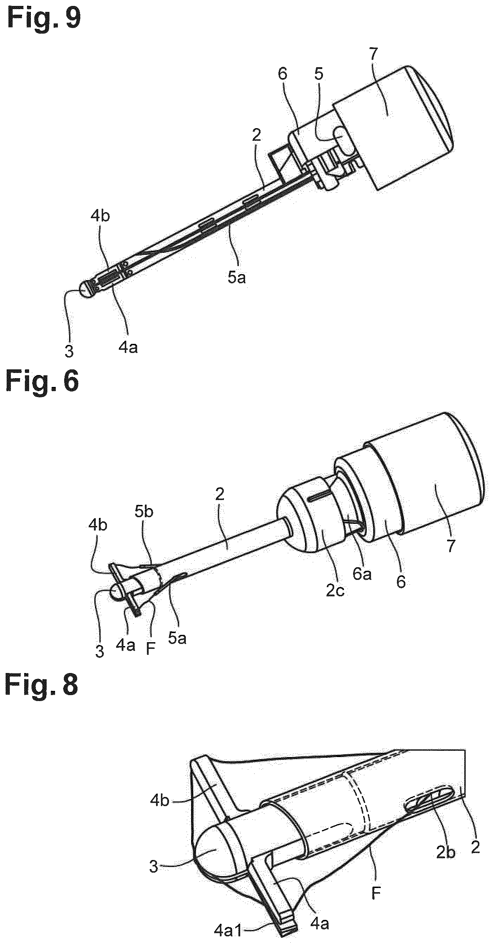

Les aiguilles (5a) et (5b) sont au nombre de deux et sont positionnées dans des logements orientés et formés dans l'épaisseur du corps (1) (figure 9). Ces logements sont donc orientés pour permettre, sous un effort de poussée des aiguilles, leur débordement angulaire de manière symétrique et leur engagement au travers d'ouvertures (2a) et (2b) que présente la canule du trocart. Les extrémités des aiguilles (5a) et (5b) sont conformées pour saisir les brins du fil (F) portés par les bras (4a) et (4b). Autrement dit, les extrémités d'accrochage des aiguilles (5a) et (5b) coopèrent avec les fentes (4a1) et (4b1) des bras (4a) et (4b). Pour permettre le déploiement angulaire des aiguilles (5a) et (5b) en dehors du corps (1) et permettre leur logement et intégration à l'intérieur dudit corps (1), ces aiguilles sont réalisées dans un alliage super élastique du type nitinol par exemple.

Les aiguilles (5a) et (5b) sont assujetties à un organe de commande actionnable à partir de l'extérieur du corps (1). Par exemple, cet organe de commande est un poussoir (7) monté avec capacité de coulissement sur la tête de manœuvre (6). Cet organe poussoir (7) coopère avec des moyens aptes à permettre le déplacement linéaire selon deux sens opposés des aiguilles correspondant soit, à leur déploiement angulaire soit, à leur attraction à l'intérieur du corps (1). Le poussoir est avantageusement assujetti à un moyen de rappel élastique en position.

La tête de manœuvre (6) recevant notamment les organes de commande (5) et (7), présente une portée mâle de centrage et d'indexation (6a) coopérant avec une portée femelle complémentaire (2c) formée à l'extrémité du trocart considérée à l'opposé de l'extrémité d'introduction de la canule (2). Par exemple, la portée (6a), de forme tronconique, présente des ailettes d'indexation angulaire coopérant avec des fentes complémentaires formées dans la portée (2c) du trocart. La tête (6) en position d'indexation de la portée (6a), vient en butée avec la portée (2c) du trocart.

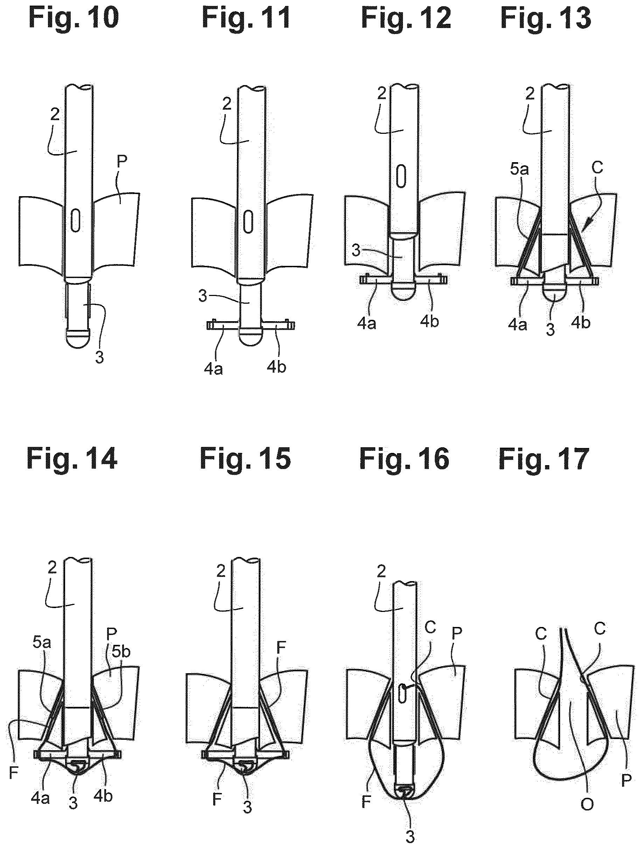

Compte tenu des caractéristiques du dispositif selon l'invention, la refermeture d'un orifice d'accès d'un instrument chirurgical est particulièrement simple sûre et efficace, évitant au chirurgien de travailler en aveugle comme c'est le cas selon l'état antérieur de la technique. A cet égard, on se réfère aux figures 10 à 17 qui montrent les différentes étapes en fin d'opération par un chirurgien, la canule (2) du trocart (T) étant introduite dans l'orifice (O) de la paroi abdominale (P), le trocart étant également équipé du système de vision.
- Le chirurgien introduit le corps tubulaire (1) dans la canule (2) du trocart jusqu'à la position de butée et d'indexation angulaire de la tête (6) par rapport à la portée (2c) du trocart. Dans cette position, l'embout (3) recevant le fil de suture (F) déborde de l'extrémité de la canule, les bras (4a) et (4b) étant en position rabattue, c'est-à- dire escamotée (figure 10).
- Le chirurgien agit sur l'organe de commande (5) pour permettre l'écartement des bras (4a) et (4b) qui se trouvent, par conséquent, disposés en alignement linéaire (figure 11). Toujours dans la position écartée des bras, le chirurgien déplace l'ensemble du trocart et du corps (1) jusqu'à ce que les bras (4a) et (4b) viennent sensiblement en butée avec la paroi abdominale (P) (figure 12).
- Le chirurgien actionne le poussoir (7) afin de provoquer le déploiement angulaire des aiguilles (5a) et (5b) qui forment, de manière concomitante de part et d'autre de l'orifice (0) et dans l'épaisseur de la paroi abdominale (P), les canaux (c) qui sont orientés angulairement et symétriquement par rapport audit orifice (0) (figure 13).
- Dans cette position, les aiguilles qui ont accroché les brins du fil (F) engagés dans les extrémités ouvertes des bras (4a) et (4b), sont ensuite rétractées par le chirurgien qui relâche le poussoir (7) afin de les escamoter de nouveau à l'intérieur du corps (1) et du trocart (figures 14 et 15).

Dans les phases illustrées figures 13, 14 et 15, la tête de manœuvre (6) n'est pas en position de butée contre la portée (2c) du trocart de sorte que l'embout (3) n'est pas en position de débordement maximum par rapport à la canule du trocart.
- Le chirurgien remet en position de butée la tête (6) avec le trocart et agit sur l'organe de commande (5) pour permettre la rétraction des bras (4a) et (4b) et la formation d'une boucle dont les brins sont engagés au travers des canaux (c) formés par les aiguilles en étant maintenues à l'intérieur du trocart au travers des ouvertures (2a) et (2b) (figure 16). Il suffit ensuite au chirurgien de retirer l'ensemble du trocart et former l'orifice laissé par le trocart du corps pour pouvoir saisir les brins et faire un nœud (figure 17).

Il apparait donc que tout le fil utilisé pour réaliser la suture est introduit à l'intérieur de la cavité du côté opposé à l'opérateur. Le chirurgien voit parfaitement l'opération de suture à réaliser étant donné qu'il peut utiliser le trocart qui a servi à l'intervention chirurgical et équipé de son système de vision.

A noter que l'embout (3), avec les bras recevant le fil est amovible, et peut présenter différentes qualités de fils afin d'être choisit au moment de sa mise en place. L'ensemble du dispositif est parfaitement étanche vis-à-vis de l'extérieur pour fermer des orifices réalisés dans des cavités pleines et sous pression.

Les avantages ressortent bien de la description.

## Revendications

1. Dispositif pour refermer un orifice d'accès formé par un instrument chirurgical dans une paroi abdominale, le dispositif comprenant :
- un corps tubulaire allongé (1) comprenant :
un embout cylindrique (3) comprenant un orifice, ledit embout cylindrique (3) présentant des bras escamotables (4a, 4b) diamétralement opposés selon les génératrices de l'embout, et
des logements formés dans l'épaisseur du corps tubulaire (1) ;
- un fil de suture (F) conditionné dans l'embout cylindrique (3), les brins du fil sortant de l'orifice de l'embout cylindrique (3), chaque brin étant porté par l'un des bras escamotables (4a, 4b) ;
- des aiguilles escamotables (5a, 5b) positionnées dans les logements et destinées à former, de part et d'autre de l'orifice d'accès, dans l'épaisseur de la paroi, des canaux orientés angulairement et symétriquement par rapport audit orifice, les aiguilles escamotables (5a, 5b) étant aptes à déborder, guidées par les logements, angulairement de manière symetrique par rapport audit corps (1), au travers de la paroi en direction des bras escamotables (4a, 4b) pour saisir chacune un brin du fil de suture (F), et aptes à retirer les brins dans les logements, formant ainsi une boucle de fil de suture ;
le dispositif étant **caractérisé en ce qu'**il comprend en outre
- un trocart (T) équipé d'un système de vision, le trocart (T) étant aptes à engager avec capacité de coulissement le corps tubulaire (1) dans sa canule de façon à ce que, une fois inséré dans la canule, l'embout cylindrique (3) du corps tubulaire (1) déborde de l'extrémité de la canule, la canule présentant des ouvertures (2a, 2b) qui sont positionnées alignées avec les logements du corps tubulaire (1) lorsqu'il est inséré dans la canule, de façon à ce que les aiguilles (5a, 5b) puissent déborder angulairement d'une manière symétrique et être retirées au travers des ouvertures (2a, 2b).

2. Dispositif selon la revendication 1 ***caractérisé* en ce que** les bras (4a) et (4b) sont articulés à l'embout cylindrique (3) pour occuper une position rabattue dans des logements formés d'une manière diamétralement opposée selon les génératrices dudit embout cylindrique (3) et occuper une position d'alignement linéaire de part et d'autre de l'embout (3) correspondant à la saisie du fil.

3. Dispositif selon la revendication 1 ***caractérisé* en ce que** les aiguilles (5a) et (5b) sont réalisées dans un alliage super élastique.

4. Dispositif selon la revendication 2 ***caractérisé* en ce que** les bras (4a) et (4b) sont assujettis à un organe de commande (5) actionnable à partir de l'extérieur du corps (1) pour occuper à volonté la position rabattue ou la position écartée en alignement linéaire.

5. Dispositif selon l'une quelconque des revendications 1, 2 et 4 ***caractérisé* en ce que** les extrémités des bras (4a) et (4b) présentent des fentes aptes à retenir les brins du fil en position écartée et à coopérer avec les aiguilles (5a) et (5b) pour saisir lesdits brins et à libérer ledit fil en position rabattue.

6. Dispositif selon la revendication 3 ***caractérisé* en ce que** les aiguilles (5a) et (5b) sont assujetties à un organe de commande (7) actionnable à partir de l'extrémité du corps pour être déplacées linéairement selon deux sens opposés correspondant soit au déplacement angulaire desdites aiguilles soit, à leur rétractation à l'intérieur dudit corps.

7. Dispositif selon la revendication 1 ***caractérisé* en ce que** l'embout cylindrique (3) recevant le fil de suture est démontable.

8. Dispositif selon l'une quelconque des revendications 1 à 7 ***caractérisé* en ce que** les organes de commande des bras (4a) et (4b) et des aiguilles (5a) et (5b) sont montés dans une tête de manœuvre (6) formée coaxialement au corps et présentant une portée axiale de centrage et de butée coopérant avec une portée femelle complémentaire que présente une extrémité du trocart considérée à l'opposé de son extrémité d'introduction.

## Patentansprüche

1. Vorrichtung zum Verschließen einer Zugangsöffnung, die durch ein chirurgisches Instrument in einer Bauchdecke gebildet wird, wobei die Vorrichtung umfasst:
- einen länglichen rohrförmigen Körper (1), umfassend:
eine zylindrische Spitze (3), die eine Öffnung umfasst, wobei die zylindrische Spitze (3) einziehbare Arme (4a, 4b) aufweist, die gemäß den Generatoren der Spitze diametral gegenüberliegen, und
Gehäuse, die in der Dicke des Rohrkörpers (1) ausgebildet sind,
- ein Nahtmaterial (F), das in der zylindrischen Spitze (3) verpackt ist, wobei die Stränge des Nahtmaterials aus der Öffnung der zylindrischen Spitze (3) herausragen, wobei jeder Strang von einem der einziehbaren Arme (4a, 4b) getragen wird ;
- einziehbare Nadeln (5a, 5b), die in den Gehäusen angeordnet sind und dazu bestimmt sind, auf beiden Seiten der Zugangsöffnung in der Dicke der Wand Kanäle zu bilden, die in Bezug auf die genannte Öffnung winklig und symmetrisch ausgerichtet sind, wobei die einziehbaren Nadeln (5a, 5b) überlaufen können, geführt durch die Gehäuse, winkelsymmetrisch in Bezug auf den Körper (1), durch die Wand zu den einziehbaren Armen (4a, 4b), um jeweils einen Faden des Fadens (F) zu greifen, und in der Lage, die Stränge in den Gehäusen zu entfernen, wodurch eine Nahtschleife gebildet wird
wobei die Vorrichtung ***dadurch gekennzeichnet* ist, dass** sie ferner umfasst
- einen Trokar (T), der mit einem Sichtsystem ausgestattet ist, wobei der Trokar (T) in der Lage ist, den Rohrkörper (1) in seine Kanüle zu schieben, so dass sich die zylindrische Spitze (3) des Rohrkörpers (1) nach dem Einsetzen in die Kanüle über das Ende der Kanüle hinaus erstreckt, die Kanüle Öffnungen (2a, 2b) aufweist, die ausgerichtet auf die Gehäuse des Rohrkörpers (1) beim Einsetzen in die Kanüle positioniert sind, so dass die Nadeln (5a, 5b) symmetrisch winklig vorstehen und durch die Öffnungen (2a, 2b) entfernt werden können.

2. Vorrichtung nach Anspruch 1, ***dadurch gekennzeichnet,* dass** die Arme (4a) und (4b) an der zylindrischen Spitze (3) angelenkt sind, um eine gefaltete Position in Gehäusen einzunehmen, die gemäß den Erzeugungen der zylindrischen Spitze (3) diametral entgegengesetzt ausgebildet sind, und um eine lineare Ausrichtungsposition auf beiden Seiten der Spitze (3) einzunehmen, die dem Greifen des Drahtes entspricht..

3. Vorrichtung nach Anspruch 1, ***dadurch gekennzeichnet,* dass** die Nadeln (5a) und (5b) aus einer superelastischen Legierung bestehen.

4. Vorrichtung nach Anspruch 2, ***dadurch gekennzeichnet,* dass** die Arme (4a) und (4b) an einem Steuerelement (5) befestigt sind, das von außerhalb des Körpers (1) betätigt werden kann, um die heruntergeklappte Position oder die Spreizposition in linearer Ausrichtung beliebig zu besetzen.

5. Vorrichtung nach einem der Ansprüche 1, 2 und 4, ***dadurch gekennzeichnet,* dass** die Enden der Arme (4a) und (4b) Schlitze aufweisen, die in der Lage sind, die Fadenstränge in einer beabstandeten Position zu halten und mit den Nadeln (5a) und (5b) zusammenzuwirken, um die Stränge zu greifen und den Faden in einer gefalteten Position zu lösen.

6. Vorrichtung nach Anspruch 3, ***dadurch gekennzeichnet,* dass** die Nadeln (5a) und (5b) an einem Steuerelement (7) befestigt sind, das vom Ende des zu bewegenden Körpers aus linear in zwei entgegengesetzte Richtungen betätigt werden kann, die entweder der Winkelverschiebung der Nadeln oder ihrem Zurückziehen innerhalb des Körpers entsprechen.

7. Vorrichtung nach Anspruch 1, ***dadurch gekennzeichnet,* dass** die zylindrische Spitze (3), die das Nahtmaterial aufnimmt, abnehmbar ist.

8. Vorrichtung nach einem der Ansprüche 1 bis 7, ***dadurch gekennzeichnet,* dass** die Steuerelemente der Arme (4a) und (4b) und der Nadeln (5a) und (5b) in einem Betätigungskopf (6) montiert sind, der koaxial zum Körper ausgebildet ist und ein axiales Zentrier- und Widerlager aufweist, das mit einem komplementären Innenlager zusammenwirkt, das ein Ende des Trokars gegenüber seinem Einsetzende betrachtet..

## Claims

1. A device for closing an access opening formed by a surgical instrument in an abdominal wall, the device comprising :
- an elongated tubular body (1) comprising :
a cylindrical tip (3) comprising an orifice, said cylindrical tip (3) having retractable arms (4a, 4b) diametrically opposed according to the generators of the tip, and
housings formed in the thickness of the tubular body (1),
- a suture (F) packaged in the cylindrical tip (3), the strands of the suture emerging from the opening of the cylindrical tip (3), each strand being carried by one of the retractable arms (4a, 4b) ;
- retractable needles (5a, 5b) positioned in the housings and intended to form, on either side of the access opening, in the thickness of the wall, channels oriented angularly and symmetrically with respect to the said opening, the retractable needles (5a, 5b) being able to overflow, guided by the housings, angularly symmetrically with respect to said body (1), through the wall towards the retractable arms (4a, 4b) to each grasp a strand of the suture (F), and able to remove the strands in the housings, thus forming a suture loop
the device being ***characterized* in that** it further comprises
- a trocar (T) equipped with a vision system, the trocar (T) being able to slide the tubular body (1) into its cannula so that, once inserted into the cannula, the cylindrical tip (3) of the tubular body (1) extends beyond the end of the cannula, the cannula having openings (2a, 2b) which are positioned in alignment with the housings of the tubular body (1) when inserted into the cannula, so that the needles (5a, 5b) can protrude angularly in a symmetrical manner and be removed through the openings (2a, 2b)

2. Device according to claim 1 ***characterized* in that** the arms (4a) and (4b) are articulated to the cylindrical tip (3) to occupy a folded position in housings formed in a diametrically opposite manner according to the generatrices of said cylindrical tip (3) and to occupy a linear alignment position on either side of the tip (3) corresponding to the gripping of the wire.

3. Device according to claim 1, ***characterized* in that** the needles (5a) and (5b) are made of a superelastic alloy.

4. Device according to claim 2, ***characterized* in that** the arms (4a) and (4b) are secured to a control element (5) actuatable from outside the body (1) to occupy at will the folded down position or the spread position in linear alignment.

5. Device according to any of claims 1, 2 and 4, ***characterized* in that** the ends of the arms (4a) and (4b) have slots capable of retaining the thread strands in a spaced position and of cooperating with the needles (5a) and (5b) to grasp said strands and to release said thread in a folded position.

6. Device according to claim 3 ***characterized* in that** the needles (5a) and (5b) are attached to a control element (7) actuable from the end of the body to be moved linearly in two opposite directions corresponding either to the angular displacement of said needles or to their retraction inside said body.

7. Device according to claim 1, ***characterized* in that** the cylindrical tip (3) receiving the suture is removable.

8. Device according to any of claims 1 to 7, ***characterized* in that** the control elements of the arms (4a) and (4b) and needles (5a) and (5b) are mounted in an operating head (6) formed coaxially with the body and having an axial centering and abutment bearing cooperating with a complementary female bearing which has an end of the trocar considered opposite its insertion end.
